# EUROPEAN PATENT SPECIFICATION

(11) **EP 4 444 858 B1**
(45) Date of publication and mention of the grant of the patent: **12.08.2026**
(21) Application number: 23721646.0
(22) Date of filing: 21.04.2023
(51) Int. Cl.: C12N 1/16, C12G 1/00, C12N 1/18

(54) **YEAST-BASED NUTRIENT AND USES THEREOF**
NÄHRSTOFF AUF HEFEBASIS UND VERWENDUNGEN DAVON
NUTRIMENT À BASE DE LEVURE ET SES UTILISATIONS

(30) Priority: 21.04.2022 EP 22305593
(43) Date of publication of application: 16.10.2024
(73) Proprietor: Danstar Ferment AG, 6300 Zug (CH)
(72) Inventor: ORTIZ-JULIEN, Anne, 31702 Blagnac (FR); FERREIRA, David, 31702 Blagnac (FR)
(74) Representative: J A Kemp LLP
(86) International application number: PCT/EP2023/060435
(87) International publication number: WO 2023/203196

(56) References cited:
- US-A1- 2008 118 600
- ANONYMOUS: "Go-Ferm Protect Evolution Technical Datasheet", LALLEMAND CATALOGUE, 1 April 2022 (2022-04-01), pages 1 - 2, XP093340412, Retrieved from the Internet <URL:https://products.lallemandwine.com/storage/files/nutrients-and-protectors/33-technical-datasheet-us-1716369903.pdf> [retrieved on 20251110]
- ANGELES POZO-BAYON M ET AL: "Scientific evidences beyond the application of inactive dry yeast preparations in winemaking", FOOD RESEARCH INTERNATIONAL, ELSEVIER, AMSTERDAM, NL, vol. 42, no. 7, 1 August 2009 (2009-08-01), pages 754 - 761, XP026131984, ISSN: 0963-9969, [retrieved on 20090401], DOI: 10.1016/J.FOODRES.2009.03.004
- VIRGINIE SOUBEYRAND ET AL: "Formation of Micella Containing Solubilized Sterols during Rehydration of Active Dry Yeasts Improves Their Fermenting Capacity", JOURNAL OF AGRICULTURAL AND FOOD CHEMISTRY, vol. 53, no. 20, 1 October 2005 (2005-10-01), pages 8025 - 8032, XP055073377, ISSN: 0021-8561, DOI: 10.1021/jf050907m
- RODRIGUEZ R J ET AL: "A requirement for ergosterol to permit growth of yeast sterol auxotrophs on cholestanol", BIOCHEMICAL AND BIOPHYSICAL RESEARCH COMMUNICATIONS, ELSEVIER, AMSTERDAM NL, vol. 106, no. 2, 31 May 1982 (1982-05-31), pages 435 - 441, XP024839001, ISSN: 0006-291X, [retrieved on 19820531], DOI: 10.1016/0006-291X(82)91129-9

## Description

### FIELD OF THE INVENTION

This specification relates to yeast-based nutrients to prevent or reduce slow and/or sluggish alcoholic fermentation and maintaining or increase the fermenting capacity of Active Dry Yeast (ADY) or Instant Dry Yeast (IDY) during alcoholic fermentation.

### BACKGROUND TO THE INVENTION

Modern fermentation processes used to produce wine, cider, beer, sake, distilled spirits and other fermentation products include the use of selected yeasts. These selected yeasts are usually made commercially available to the producer in dry forms, either as vermicelli or pellets (Instant Dry Yeast IDY and Active Dry Yeast ADY). These dry forms are standard in the yeast industry as they allow long term storage while keeping a very high viability in the range 1 to 4 x 10¹⁰ colony forming units per gram of dry products. Commercial dry yeasts include more than 500 different strains. Most of them belong to *Saccharomyces* genus and a few to other genii such as *Torulaspora, Metschnikowia, Pichia, Lachancea, Starmerella, Kluyveromyces or Candida.*

The use of a selected yeast strain is an essential quality step in modern winemaking and other fermentation industries. For winemaking, grapes are crushed or pressed to release a grape must. As harvested grapes are not pasteurised and sterilized, they contain a vast population of indigenous (or wild) yeasts of different genii. Yeast cells of the selected commercial strain compete with this indigenous flora. It is therefore essential for the winemaker to ensure a "successful implantation" of the selected yeast strain. This means that the selected yeast strain must be added with adequate viability so that it becomes predominant among naturally occurring wild yeasts in the fermentation media. It is accepted in the fermentation industry, and especially in the winemaking industry, that such selected yeasts must be added after a careful rehydration step. The objective of rehydration is to achieve the maximum viability and yeast activity expressed as the percentage of live cells (vitality per cell) immediately prior to introduction of the rehydrated yeast into the grape must. Rehydration is dealt with extensively in the article entitled "Rehydration Protocols for Active Dry Wine Yeasts and the Search for Early Indicators of Yeast Activity" published in Am. J. Enol. Vitic. 57 :4 (2006).

Standard rehydration generally includes the following steps :
- Preparation of a water solution at 37 to 40 ° Celsius in a rehydration vessel (a bucket or a small tank). Optionally, sugar or grape must can be added to reach 50 g/l of sugar;
- Addition of ADY or IDY to this warm solution with continuous stirring;
- mixing (gentle to vigorous);
- the solution stands for about 20 min to achieve rehydration. Formation of foam due to the production of CO₂ can be visible at this stage (depending on the strain)
- preferably the solution is diluted progressively using grape must from the vessel to be fermented. This is to avoid a temperature shock if there is a large difference with respect to the cold must (this step may require additional time);
- addition to fermentation tank (the temperature of the must is generally in the range 10-25 °C at this stage).

Another well-known critical moment of fermentation remains the end of the alcoholic fermentation. It is essential for the fermenting inoculated yeasts to be able to achieve the consumption of all sugars, avoiding sluggish or stuck fermentations. Indeed, those uncompleted fermentations represent risky situations, when other micro-organisms (spoilage microorganisms) ,or lactic acid bacteria are capable in these circumstances to colonise rapidly the fermentation medium stopping definitively the alcoholic fermentation. Some of these micro-organisms will use the residual sugars to produce undesirable metabolites such as, for example, acetic acid, resulting in losses of quality of the wines. Problematic alcoholic fermentation means in the context of the present specification fermentations that are slow fermentations and/or sluggish. The fermentation speed of a yeast in a medium is defined as the quantity of carbon dioxide released per time unit, generally expressed in grams per liter and per time unit. It directly correlated with the sugar consumption by the yeast. It is represented by the curve derived from the quantity of carbon dioxide released as a function of time V=dCO₂/dt. In the case of slow fermentations, the maximum observed speeds during the fermentation are low. These slow fermentations are generally attributable to yeast populations which have low metabolic activity, in general associated with a deficiency of assimilable nitrogen in the musts. Sluggish fermentations or fermentation stoppages are characterized by a maximum fermentation speed which is relatively high but which diminishes progressively, the viability and vitality of the yeasts becoming very low. These fermentations slow down significantly then or stop totally when the yeast population is insufficient to ensure the complete consumption of the sugars. In most cases, this type of phenomenon is observed when there is sterols or oxygen deficiencies or an imbalance between lipids and nitrogen content: low lipids associated with high nitrogen levels.

It is well known that these problematic fermentations are most frequently associated with imbalances or deficiencies of the nutritive media (Alexandre et al. J. of Ind. Microbiol. and Biotechnology, vol. 20, 1998: pages 20-27). Indeed, different nutrients are necessary to allow yeasts on the one hand to develop sufficiently well to colonise the fermentation medium and on the other hand to ensure effectively the metabolism of the sugars in alcohol, and this to the point of total exhaustion of these sugars. These nutrients belong especially to the following categories: sources of nitrogen, sources of lipids, vitamins, mineral salts.

EP1395649B2 describes the rehydration of active dry yeasts (ADYs) in a medium with an added nutritive complex comprising yeast derivatives and optionally other nutrients to increase the fermentation capacity of the yeast produced from this rehydration step and keep it until the end of the alcoholic fermentation.

EP1814978A2 (corresponding to US2008 /118600) describes an improvement of the rehydration of active dry yeasts (ADYs) in a medium, the rehydration medium containing inactivated yeasts with a high content of certain types of sterols, either naturally, or by enrichment, or by using sterol compositions in soluble form.

Ángeles Pozo-Bayón, M., et al. (2009), Food Research International 42(7):754-761 describes scientific evidences beyond the application of inactive dry yeast preparations in winemaking.

Soubeyrand, V., et al. (2005), Journal of Agricultural and Food Chemistry 53(20):8025-32 describes the formation of micella containing solubilized sterols during rehydration of active dry yeasts, and the effect on their fermenting capacity.

Rodriguez, RJ., et al. (1982) Biochemical and biophysical research communications 106(2):435-441 describes a requirement for ergosterol to permit growth of yeast sterol auxotrophs on cholestanol.

Go-Ferm Protect Evolution Technical Datasheet, LALLEMAND CATALOGUE, 1 April 2022, is a technical datasheet concerning the product Go-Ferm Protect Evolution^{™}.

It would be highly desirable to be provided with a more versatile yeast-based nutrients having the same capability to prevent or reduce slow and/or sluggish alcoholic fermentation and maintain or increase the fermenting capacity of Active Dry Yeast (ADY) or Instant Dry Yeast (IDY) during alcoholic fermentation.

### SUMMARY OF THE INVENTION

The scope of the invention is defined by the claims. Any aspects and examples of the present disclosure which do not fall under the scope of the appended claims do not form part of the invention and are merely provided for illustrative purposes.

The invention provides a yeast-based nutrient having a sterol content of at least 20 mg per gram of yeast-based nutrient, wherein the yeast-based nutrient is in a microgranules form and is capable of maintaining or increasing fermenting capacity of an Active Dry Yeast (ADY) or an Instant Dry Yeast (IDY) during alcoholic fermentation, wherein the microgranules have a median particle size, on a particle volume basis, of from about 200 µm to about 400 µm, and wherein the yeast-based nutrient is an inactivated yeast, a yeast autolysate, a yeast cell hull, yeast extract or mixtures thereof.

The invention also provides a method of rehydrating Active Dry Yeast (ADY) or Instant Dry Yeast (IDY), wherein the method comprises adding a yeast-based nutrient having a sterol content of at least 20 mg per g of the yeast-based nutrient and in a microgranules form to the ADY or IDY in a rehydration media at less than 37°C, wherein the microgranules have a median particle size, on a particle volume basis, of from about 200 µm to about 400 µm, and wherein the yeast-based nutrient is an inactivated yeast, a yeast autolysate, a yeast cell hull, yeast extract or mixtures thereof.

The invention also provides a method of maintaining or increasing a fermenting capacity of Active Dry Yeast (ADY) or Instant Dry Yeast (IDY) during alcoholic fermentation, the method comprises adding a yeast-based nutrient having a sterol content of at least 20 mg per gram of the yeast-based nutrient in a rehydration media with the Active Dry Yeast (ADY) or Instant Dry Yeast (IDY) to be rehydrated, or in a vessel containing the Active Dry Yeast (ADY) or Instant Dry Yeast (IDY) and a must to be fermented, wherein the yeast-based nutrient is in a microgranules form and is capable of increasing or maintaining a fermenting capacity of Active Dry Yeast (ADY) or Instant Dry Yeast (IDY) during alcoholic fermentation, wherein the microgranules have a median particle size, on a particle volume basis, of from about 200 µm to about 400 µm, and wherein the yeast-based nutrient is an inactivated yeast, a yeast autolysate, a yeast cell hull, yeast extract or mixtures thereof.

### BRIEF DESCRIPTION OF THE DRAWINGS

For a better understanding of the disclosure, and to show how the same may be carried into effect, reference will now be made, by way of example, to the accompanying drawings in which;
Figure 1 compares the duration of different alcoholic fermentation assays F1 to F9 carried out with yeast LALVIN QA 23 YSEO^{®} (QA) , rehydrated at 20 or 37°C or added directly (DI) to the Sauvignon must in a dosage of 25g/hl and in the presence or the absence of Yeast-based Nutrient; and
Figure 2 compares the viability in CFU/ml at 144 hours in the of the same alcoholic fermentation assays of Figure 1 (F1 to F9); and
Figure 3 compares the Volatile Acidity of the wines resulting from the alcoholic fermentation assays F1 to F9 of Figs. 1 and 2; and
Figure 4 illustrates the transfer of sterols from a yeast-based nutrient to live yeast (ADYs and IDYs); and
Figure 5 compares the duration of different alcoholic fermentation assays of a Chardonnay must carried out with yeast LALVIN CY3079^{™} in a dosage of 25g/hl, the LALVIN CY3079^{™} was rehydrated at 20 or 37°C and in the presence or the absence of Yeast-based Nutrient; and
Figure 6 compares the duration of different alcoholic fermentation assays of a Chardonnay must carried out with yeast LALVIN CY3079^{™} in a dosage of 25g/hl, the LALVIN CY3079^{™} was rehydrated at 20 or 37°C and in the presence or the absence of Yeast-based Nutrient.

### DETAILED DESCRIPTION

Slow and/or sluggish alcoholic fermentation and issues with the fermenting capacity of Active Dry Yeast (ADY) or Instant Dry Yeast (IDY) during alcoholic fermentation has always been an issue for winemakers. It is well known that sterols are important constituents of the cell membrane of yeasts. The sterol concentration should be optimal to ensure the integrity of the membrane, which is a guarantee of activity of the membrane proteins, such as permeases. Under fermenting conditions, if some of these permeases no longer function, a drop in cell vitality is observed, which may result in the death of the cell. The sterol composition of yeasts thus plays a fundamental role in their fermenting capacity.

It is believed that yeast (ADYs and IDYs) with high sterol content during the alcoholic fermentation is achieved by means of transferring and incorporating the sterols in the form of micelles from a yeast-based nutrient in accordance with the present description. In addition, this high sterol content reduces the production of volatile acids, which makes it possible to improve the organoleptic properties of the fermentation products.

The present disclosure provides a yeast-based nutrients capable to prevent or reduce slow and/or sluggish alcoholic fermentation and also to increase or improve the fermenting capacity of yeast (Active Dry Yeast (ADY) or Instant Dry Yeast (IDY)) during alcoholic fermentation.

The term "yeast (Active Dry Yeast (ADY) or Instant Dry Yeast (IDY))" when used herein means a yeast strain of the genii *Saccharomyces, Torulaspora, Metschnikowia, Pichia, Lachancea, Starmerella, Kluyveromyces* or *Candida,* or a mixture thereof capable of performing an alcoholic fermentation and able to reproduce under favorable conditions while actively fermenting.

In the context of the present disclosure, a "yeast-based nutrient" means a nutrient made from or contains inactivated yeasts, yeast autolysates, yeast cell hulls, yeast cell hulls, yeast extracts or mixtures thereof issued from yeasts naturally rich in sterols or yeasts that have been enriched in sterols during its propagation, especially in ergosterol and/or zymosterol and/or ergosta-5,7-dienols, advantageously according to the techniques known to those skilled in the art. The yeasts naturally rich in sterols or yeasts that have been enriched in sterols are from genii Saccharomyces, *Torulaspora, Metschnikowia, Pichia, Lachancea, Starmerella, Kluyveromyces* or *Candida,* or a mixture thereof.

Yeasts that have been enriched in sterols are cultured under a constant and high aeration. The propagation temperature is from about 15 to 40° C. and preferably from 25 to 35° C. The yeasts are cultured by constant supply of carbon sources and nitrogen sources, and also by an intermittent supply of essential growth factors. The carbon sources are generally derived from molasses and may be sugar or beetroot molasses or a mixture thereof in variable concentrations. The nitrogen source may originate from various ammoniacal derivatives, for instance ammonium hydroxide, ammonium chloride, ammonium sulfate, ammonium phosphate, diammonium phosphate or crude protein extracts. The yeast propagation is performed according to a program comprising growth rate variations. The maximum growth rates are from 0.05/h to 0.25/h and preferably from 0.15 to 0.2/h. The sterol enrichment of the yeast can be performed by specifically modifying the carbon and nitrogen sources so as to reduce the total level of nitrogen relative to that of carbon. It is also possible to add specific sterol precursors in a controlled manner. Preferred sources of sterol precursors include ethanol, acetic acid, squalene and lanosterol. These precursors may be added in purified or crude form.

Alternatively, the sterol enrichment of the yeast-based nutrient can be done using a lipid composition comprising sterols. The lipid composition comprising sterols can be mixed with inactivated yeasts, yeast autolysates, yeast cell hulls, yeast extracts or mixtures thereof. The lipid composition comprises for example ergosterol, zymosterol and a lipopolysaccharide-protein complex that can be precipitated. These lipids compositions advantageously contain from 10% to 70% by dry weight of ergosterol and from 10% to 70% by dry weight of zymosterol, in a proportion of from 10 to 20 mg of total sterols per g and from 500 to 1000 mg/g of lipopolysaccharide-protein complex. Advantageously, the sterol fraction also comprises one or more sterols such as fecosterol, lanosterol, ergosta-5,7-dienols (dihydroergosterol, methyl-zymosterol, demethyllanosterol and ergosta-7,22-dien-3-ol).

It was unexpectedly found that the yeast-based nutrient naturally rich or enriched in sterols of the present description, when in microgranules form, allows for a significant shorter fermentation time, while still capable of preventing or reducing slow and/or sluggish alcoholic fermentation and maintaining or increasing the fermenting capacity of Active Dry Yeast (ADY) or Instant Dry Yeast (IDY) during alcoholic fermentation than similar products in powder form for example.

The yeast-based nutrient may have a sterol content of at least 20 mg per gram of yeast-based nutrient, in a microgranules form and is capable of maintaining or increasing a fermenting capacity of Active Dry Yeast (ADY) or Instant Dry Yeast (IDY) during alcoholic fermentation. The sterol content may be at least 21 mg, 22 mg, 23 mg, 24 mg, 25 mg, 26 mg, 27 mg, 28 mg, 29 mg, 30 mg, 31 mg, 32 mg, 33 mg, 34 mg, 35 mg per gram of yeast-based nutrient. The sterol content may be within the range of about 20 mg to 35 mg per gram of nutrient. The sterol content may be within the range of about 25 mg to 35 mg per gram of nutrient. The yeast-based nutrient may be naturally rich in sterols or is enriched in sterols. Alternatively, the yeast-based nutrient may be added a to sterol composition in soluble form.

The microgranules of the present disclosure can be obtained according to methods known to the skilled person in the art. The microgranules in accordance with the present description may have a particle size smaller than 500 µm. The microgranules may have a median particle size (D₅₀), on a particle volume basis, of from about 200 µm to about 400 µm. The microgranules may have a median particle size (D50), on a particle volume basis, of from about 200 µm to about 300 µm.

The yeast-based nutrient may be from any yeast sp., such as, for example, *Saccharomyces, Torulaspora, Metschnikowia, Pichia, Lachancea, Starmerella, Kluyveromyces* or *Candida .* The yest-based nutrient may be from a mixture of at least two of *Saccharomyces, Torulaspora, Metschnikowia, Pichia, Lachancea, Starmerella, Kluyveromyces* or *Candida.*

The yeast-based nutrient may be in the form of an inactivated yeast, a yeast autolysate, a yeast cell hull, yeast extract or mixtures thereof. The yeast-based nutrient may be in the form of an inactivated yeast. The yeast-based nutrient may be in the form of a yeast autolysate.

The yeast-based nutrient in microgranules form may be added to a rehydration media of yeast (ADY or IDY) to be rehydrated according to methods known to the skilled person in the art. It was unexpectedly found that the yeast-based nutrient in microgranules form permits a rehydration without having to heat the rehydration media to 37 degrees Celsius. In other words, a rehydration made with the yeast-based nutrient in microgranules form in accordance with the present disclosure can be made at room temperature or at temperature between about 15 and about 25 degrees Celsius. Performing a rehydration at room temperature or between about 15 and about 25 degrees Celsius provides the advantage of not requiring access to hot water, therefore reducing the number of steps. Advantageously, the resulting rehydration medium can be used to inoculate the fermentation vessel at the temperature of the must which is generally in the range of about 10 to about 25 °Celsius, without the need to dilute progressively the rehydration medium using grape must from the tank vessel to be fermented, avoiding the possibility of a temperature shock if there is a large difference with respect to the cold must to be fermented. The yeast-based nutrient in microgranules form also allows for a greater solubility in the rehydration medium.

Alternatively, the yeast-based nutrient in microgranules form may be added directly to a vessel containing the yeast (ADY or IDY) and a must to be fermented. Performing a direct addition of the yeast-based nutrient in microgranules form provides the advantages of simplifying the steps, especially in large wineries, such as: no requirement to have access to hot water and the yeast-based nutrient in microgranules form can be used at the temperature of the must which is generally in the range of about 10 to about 25 °Celsius. The yeast-based nutrient in microgranules form also allows for a greater solubility in the must.

The present disclosure also provides a method of maintaining or increasing a fermenting capacity of Active Dry Yeast (ADY) or Instant Dry Yeast (IDY) during alcoholic fermentation. The method comprises adding a yeast-based nutrient having a sterol content of at least 20 mg per g of the yeast-based nutrient in a rehydration media of yeast (ADY or IDY) to be rehydrated. Alternatively, the method comprises adding a yeast-based nutrient having a sterol content of at least 20 mg per g of the yeast-based nutrient in a fermentation vessel containing the yeast (ADY or IDY) and a must to be fermented. The yeast-based nutrient may be in a microgranules form and may be capable of increasing or maintaining a fermenting capacity of Active Dry Yeast (ADY) during alcoholic fermentation.

The method may comprise adding a yeast-based nutrient having a sterol content of at least 20 mg per gram of the yeast-based nutrient in a rehydration media with the Active Dry Yeast (ADY) or Instant Dry Yeast (IDY) to be rehydrated, or in a vessel containing the Active Dry Yeast (ADY) or Instant Dry Yeast (IDY) and a must to be fermented, wherein the yeast-based nutrient is in a microgranules form and is capable of increasing or maintaining a fermenting capacity of Active Dry Yeast (ADY) or Instant Dry Yeast (IDY) during alcoholic fermentation. The ADY or IDY may be in powder, vermicelli or pellet form. The sterol content may be at least 21 mg, 22 mg, 23 mg, 24 mg, 25 mg, 26 mg, 27 mg, 28 mg, 29 mg, 30 mg, 31 mg, 32 mg, 33 mg, 34 mg, 35, mg per gram of yeast-based nutrient. The sterol content may be within the range of about 20 mg to 35 mg per gram of nutrient. The sterol content may be within the range of about 25 mg to 35 mg per gram of nutrient. The yeast-based nutrient may be naturally rich in sterols or is enriched in sterols. Alternatively, the yeast-based nutrient may be added to a sterol composition in soluble form. The microgranules may have a size smaller than 500 µm. At least 50% of the microgranules may have a size within the range of about 200 to about 400 µm. At least 50% of the microgranules may have a size within the range of about 200 to about 300 µm. The yeast-based nutrient may be from any yeast sp., such as, for example, Saccharomyces, *Torulaspora, Metschnikowia, Pichia, Lachancea, Starmerella, Kluyveromyces or Candida* The yest-based nutrient may be from a mixture of at least two of *Saccharomyces, Torulaspora, Metschnikowia, Pichia, Lachancea, Starmerella, Kluyveromyces* or *Candida.*

The method may comprise rehydrating the Active Dry Yeast (ADY) or Instant Dry Yeast (IDY), comprising adding a yeast-based nutrient having a sterol content of at least 20 mg per g of the yeast-based nutrient and in a microgranules form to the ADY or IDY in a rehydration media at less than 37°C. The rehydration may be performed for 20 minutes or less. The rehydration media may be water. The rehydration temperature may be 30°C or less, such as 25°C or less, or 20°C or less. The rehydration temperature may be 10°C - 30°C, such as 10°C - 25°C, 10°C - 20°C, 15°C - 30°C, 15°C-25°C or 15°C-20°C. The rehydration temperature may be 10°C - 25°C, such as 15°C-25°C or 15°C-20°C. The rehydration may be performed for 19 minutes or less, 18 minutes or less, 17 minutes or less, 16 minutes or less, 15 minutes or less, 10 minutes or less or 5 minutes or less. The rehydration may be performed for 1-20 minutes, such as 2-20 minutes, 3-20 minutes, 4-20 minutes, 5-20 minutes, 1-15 minutes, 2-15 minutes, 3-15 minutes, 4-15 minutes or 5-15 minutes. The rehydration temperature may be 10°C - 25°C, such as 15°C-25°C or 15°C-20°C, and the rehydration is performed for 15 minutes or less, such as 5-15 minutes. The characteristics of the yeast-based nutrient may be as described above. The method may further comprise performing an alcoholic fermentation of a fermentation medium. The fermentation medium may be a wine must. The fermentation may be performed at 10°C - 25°C.

The yeast-based nutrient may be in the form of an inactivated yeast, a yeast autolysate, a yeast cell hull, yeast extract or mixtures thereof. The yeast-based nutrient may be in the form of an inactivated yeast. The yeast-based nutrient may be in the form of a yeast autolysate.

The present disclosure will now be explained in more detail with reference to the following winemaking examples. For comparison purposes, the method of the present disclosure and a standard rehydration process were used.

### EXAMPLE 1: Maintain or increase of the fermentation profile of a Sauvignon must by the incorporation of a yeast-based nutrient in accordance with the present description

Fermentations were carried out in parallel and in duplicate on a Sauvignon must coming from South-West region of France and containing 200 mg/l of assimilable nitrogen and containing 250 g/l of fermentable sugars, 30NTU, a pH of 3,18, and a Total acidity of 4,50 g H2SO4/L. The active dry yeast used is the commercial yeast LALVIN QA 23 YSEO^{®} and is inoculated in a dosage of 25g/hl. The yeast-based nutrients were added in a dosage of 30g/hl.

Rehydration and direct inoculation condition:
20 mins rehydration at 20 or 37°C:
   2.5 grams of the dry yeast LALVIN QA 23 YSEO^{®} is rehydrated in 40 ml of water at 20 or 37°C for 20 minutes with 3 grams of Yeast-based Nutrient. In the control rehydration, no addition of Yeast-based Nutrient is made.
2 mins rehydration at 20 or 37°C:
   0,25 gram of the dry yeast LALVIN QA 23 YSEO^{®} is rehydrated in 3 ml of water at 20 or 37°C for 20 minutes with 0,3 gram of Yeast-based Nutrient. In the control rehydration, no addition of Yeast-based Nutrient is made.

Direct inoculation:
0.25 gram of LALVIN QA 23 YSEO^{®} and 0,3 gram of Yeast-based Nutrient are added directly to the Sauvignon must to be fermented. In the control direct inoculation, no addition of Yeast-based Nutrient is made.

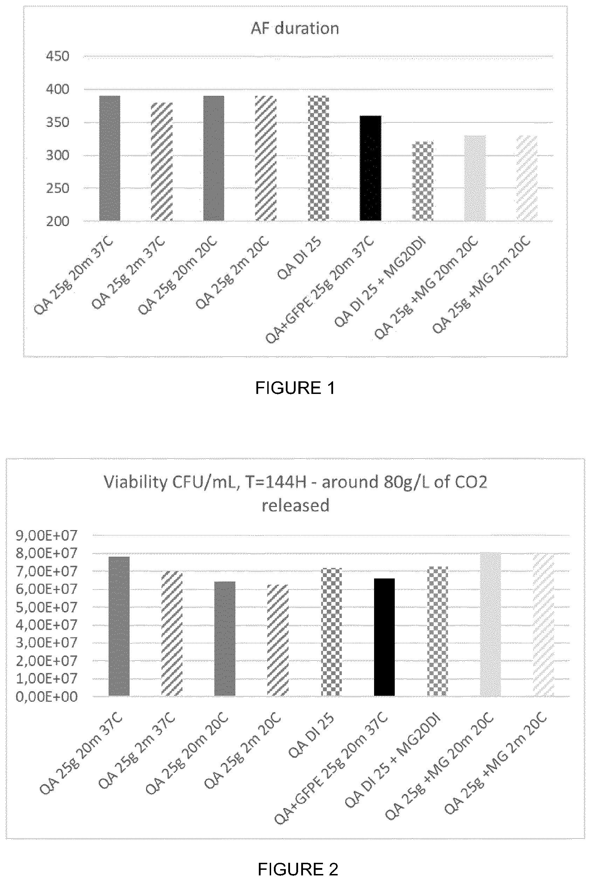

Fermentation:
The fermentations correspond to the following forms:
F1: Fermentation carried out with the yeast LALVIN QA 23 YSEO^{®} rehydrated in 40 ml of water at 37°C for 20 minutes in the absence of Yeast-based Nutrient (Figure 1 QA 25g 20m 37C).
F2: Fermentation carried out with the yeast LALVIN QA 23 YSEO^{®} rehydrated in 3 ml of water at 37°C for 2 minutes in the absence of Yeast-based Nutrient (Figure 1 QA 25g 2m 37C).
F3: Fermentation carried out with the yeast LALVIN QA 23 YSEO^{®} rehydrated in 40 ml of water at 20°C for 20 minutes in the absence of Yeast-based Nutrient (Figure 1 QA 25g 20m 20C).
F4: Fermentation carried out with the yeast LALVIN QA 23 YSEO^{®} rehydrated in 3 ml of water at 20°C for 2 minutes in the absence of Yeast-based Nutrient (Figure 1 QA 25g 2m 20C).
F5: Fermentation carried out with the yeast LALVIN QA 23 YSEO^{®} added directly to the Sauvignon must at 20°C in a dosage of 25g/hl and in the absence of Yeast-based Nutrient (Figure 1 QA DI 25g).
F6: Fermentation carried out with the yeast LALVIN QA 23 YSEO^{®} rehydrated in the presence of Goferm Protect Evolution^{™} (used in a dosage permitting a concentration of 30 g/hl in the must) in the rehydration medium (Figure 1 QA+GFPE 25g 20m 37C).
F7: Fermentation carried out with the yeast LALVIN QA 23 YSEO^{®} added directly to the Sauvignon must at 20°C in a dosage of 25g/hl and in the presence of YBN01 in a dosage of 30g/hl (Figure 1 QA DI 25g + YBN01 DI 20C).
F8: Fermentation carried out with the yeast LALVIN QA 23 YSEO^{®} rehydrated in 40 ml of water at 20°C for 20 minutes in the presence of YBN01 in a dosage of 30 g/hl (Figure 1 QA 25g + YBN01 20m 20C).
F9: Fermentation carried out with the yeast LALVIN QA 23 YSEO^{®} rehydrated in 3 ml of water at 20°C for 2 minutes in the presence of YBN01 in a dosage of 30 g/hl (Figure 1 QA 25g + YBN01 2m 20C).

The fermenters of 1 liter are inoculated with X ml of rehydration solution containing the yeast LALVIN QA 23 YSEO^{®}, corresponding to a utilization dosage in the must of 25 g/hl of active dry yeasts. The fermentation temperature is 20°C. For the direct inoculations, the 1-liter fermenters were inoculated directly with the yeast LALVIN QA 23 YSEO^{®} in a dosage of 25g/hl either in the absence of Yeast-based Nutrient or in the presence of Yeast-based Nutrient in a dosage of 30 g/hl. The production of CO2 increases as a function of time.

As shown in Figure 1, the alcoholic fermentations carried out in the presence of Yeast-based Nutrient in accordance with the present description (F7 to F9), either via direct addition or addition of rehydrated medium, are completed at least 60 hours before the control fermentations F1 to F5. Moreover, Figure 1 shows that alcoholic fermentations F7 to F9 carried out in the presence of Yeast-based Nutrient in accordance with the present disclosure (YBN01), are completed at least 30 h before the fermentation F6 carried out in the presence of Goferm Protect Evolution^{™} (by Lallemand Inc.), a previous generation Yeast-based Nutrient in powder form disclosed in European Patent No.:1395649B2.

Figure 2 shows the viability in CFU/ml at 144 hours in the fermentation and suggests that fermentations F7 to F9 exhibit a similar or better viability of the yeast LALVIN QA 23 YSEO^{®} in the presence of Yeast-based Nutrient in accordance with the present description.

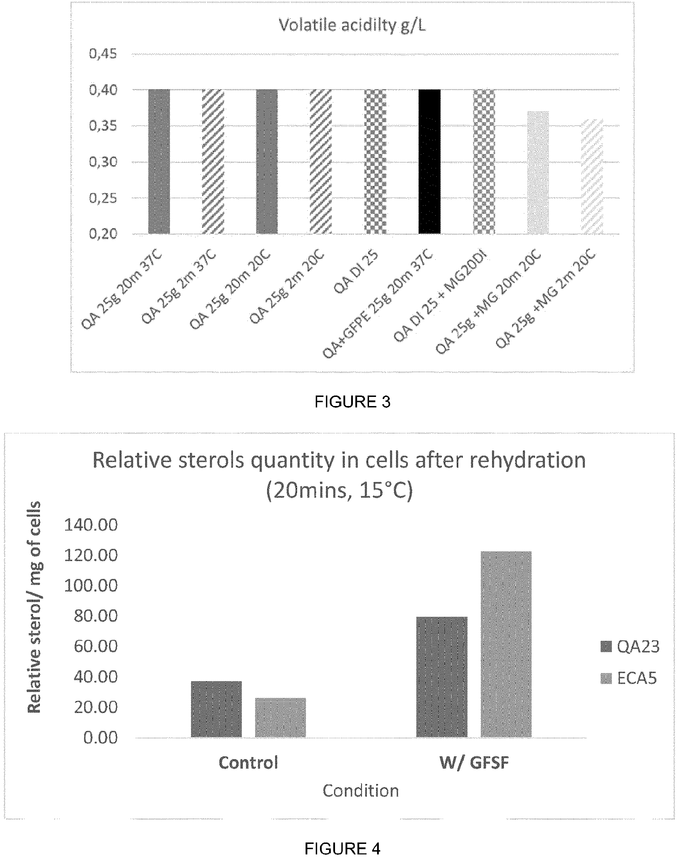

As shown in Figure 3, the Volatile acidity of the wines resulting from the fermentation F8 and F9 in the presence of Yeast-based Nutrient in accordance with the present description exhibit lower levels of Volatile acid in wine produced. The fermentation F7 (direct addition) exhibit a similar level of Volatile acid in wine produced than controls, suggesting that the direct addition of Yeast-based Nutrient in accordance with the present description is not increasing the levels of Volatile acid in wines.

The yeast-based nutrient naturally rich or enriched in sterols of the present description, when in microgranules form, allows for a significant shorter fermentation time, therefore preventing or reducing slow and/or sluggish alcoholic fermentation and maintaining or increasing the same fermenting capacity of Active Dry Yeast (ADY) or Instant Dry Yeast (IDY) during alcoholic fermentation than similar products in powder form for example. The fermentation results shows that the microgranule form of the yeast-based nutrient of the present description is very versatile and allows for utilization either in direct inoculation or in standard rehydration. Moreover, it was shown in the present description that when used in rehydration or in direct inoculation, the yeast-based nutrient of the present description does not require access to hot water and can be used at the temperature of the must which is generally in the range of about 10 to about 25 °Celsius.

### EXAMPLE 2: Maintain or increase of the fermentation profile of a Sauvignon must by the incorporation of a yeast-based nutrient

Fermentations were carried out in parallel and in duplicate on a Sauvignon must coming from South-West region of France and containing 200 mg/l of assimilable nitrogen and containing 250 g/l of fermentable sugars, 30NTU, a pH of 3,18, and a Total acidity of 4,50 g H2SO4/L. The active dry yeast used is the commercial yeast LALVIN QA 23 YSEO^{®} and is inoculated in a dosage of 25g/hl. The yeast-based nutrients were added in a dosage of 30g/hl. The yeast-based nutrients had a sterol content of at least 20 mg per gram of yeast-based nutrient and was in a microgranules form.

Rehydration and direct inoculation condition:
20 mins rehydration at 20 or 37°C:
   2.5 grams of the dry yeast LALVIN QA 23 YSEO^{®} is rehydrated in 40 ml of water at 20 or 37°C for 20 minutes with 3 grams of Yeast-based Nutrient. The yeast-based nutrients had a sterol content of at least 20 mg per gram of yeast-based nutrient and was in a microgranules form. In the control rehydration, no addition of Yeast-based Nutrient is made.
2 mins rehydration at 20 or 37°C:
   0,25 gram of the dry yeast LALVIN QA 23 YSEO^{®} is rehydrated in 3 ml of water at 20 or 37°C for 20 minutes with 0,3 gram of Yeast-based Nutrient. The yeast-based nutrients had a sterol content of at least 20 mg per gram of yeast-based nutrient and was in a microgranules form. In the control rehydration, no addition of Yeast-based Nutrient is made.

Direct inoculation:
0.25 gram of LALVIN QA 23 YSEO^{®} and 0,3 gram of Yeast-based Nutrient are added directly to the Sauvignon must to be fermented. The yeast-based nutrients had a sterol content of at least 20 mg per gram of yeast-based nutrient and was in a microgranules form. In the control direct inoculation, no addition of Yeast-based Nutrient is made.

### Fermentation:

The fermentations correspond to the following forms:
F1: Fermentation carried out with the yeast LALVIN QA 23 YSEO^{®} rehydrated in 40 ml of water at 37°C for 20 minutes in the absence of Yeast-based Nutrient (Figure 1 QA 25g 20m 37C).
F2: Fermentation carried out with the yeast LALVIN QA 23 YSEO^{®} rehydrated in 3 ml of water at 37°C for 2 minutes in the absence of Yeast-based Nutrient (Figure 1 QA 25g 2m 37C).
F3: Fermentation carried out with the yeast LALVIN QA 23 YSEO^{®} rehydrated in 40 ml of water at 20°C for 20 minutes in the absence of Yeast-based Nutrient (Figure 1 QA 25g 20m 20C).
F4: Fermentation carried out with the yeast LALVIN QA 23 YSEO^{®} rehydrated in 3 ml of water at 20°C for 2 minutes in the absence of Yeast-based Nutrient (Figure 1 QA 25g 2m 20C).
F5: Fermentation carried out with the yeast LALVIN QA 23 YSEO^{®} added directly to the Sauvignon must at 20°C in a dosage of 25g/hl and in the absence of Yeast-based Nutrient (Figure 1 QA DI 25g).
F6: Fermentation carried out with the yeast LALVIN QA 23 YSEO^{®} rehydrated in the presence of Goferm Protect Evolution^{™} (used in a dosage permitting a concentration of 30 g/hl in the must) in the rehydration medium (Figure 1 QA+GFPE 25g 20m 37C).
F7: Fermentation carried out with the yeast LALVIN QA 23 YSEO^{®} added directly to the Sauvignon must at 20°C in a dosage of 25g/hl and in the presence of YBN01 in a dosage of 30g/hl (Figure 1 QA DI 25g + YBN01 DI 20C). YBN01 was a yeast-based nutrient having a sterol content of at least 20 mg per gram of yeast-based nutrient and was in a microgranules form.
F8: Fermentation carried out with the yeast LALVIN QA 23 YSEO^{®} rehydrated in 40 ml of water at 20°C for 20 minutes in the presence of YBN01 in a dosage of 30 g/hl (Figure 1 QA 25g + YBN01 20m 20C). YBN01 was a yeast-based nutrient having a sterol content of at least 20 mg per gram of yeast-based nutrient and was in a microgranules form.
F9: Fermentation carried out with the yeast LALVIN QA 23 YSEO^{®} rehydrated in 3 ml of water at 20°C for 2 minutes in the presence of YBN01 in a dosage of 30 g/hl (Figure 1 QA 25g + YBN01 2m 20C). YBN01 was a yeast-based nutrient having a sterol content of at least 20 mg per gram of yeast-based nutrient and was in a microgranules form.

The fermenters of 1 liter are inoculated with X ml of rehydration solution containing the yeast LALVIN QA 23 YSEO^{®}, corresponding to a utilization dosage in the must of 25 g/hl of active dry yeasts. The fermentation temperature is 20°C. For the direct inoculations, the 1-liter fermenters were inoculated directly with the yeast LALVIN QA 23 YSEO^{®} in a dosage of 25g/hl either in the absence of Yeast-based Nutrient or in the presence of Yeast-based Nutrient in a dosage of 30 g/hl. The yeast-based nutrients had a sterol content of at least 20 mg per gram of yeast-based nutrient and was in a microgranules form. The production of CO2 increases as a function of time.

As shown in Figure 1, the alcoholic fermentations carried out in the presence of Yeast-based Nutrient (F7 to F9), either via direct addition or addition of rehydrated medium, are completed at least 60 hours before the control fermentations F1 to F5. The yeast-based nutrient had a sterol content of at least 20 mg per gram of yeast-based nutrient and was in a microgranules form. Moreover, Figure 1 shows that alcoholic fermentations F7 to F9 carried out in the presence of Yeast-based (YBN01), are completed at least 30 h before the fermentation F6 carried out in the presence of Goferm Protect Evolution^{™} (by Lallemand Inc.), a previous generation Yeast-based Nutrient in powder form disclosed in European Patent No.:1395649B2. YBN01 was a yeast-based nutrient having a sterol content of at least 20 mg per gram of yeast-based nutrient and was in a microgranules form.

Figure 2 shows the viability in CFU/ml at 144 hours in the fermentation and suggests that fermentations F7 to F9 exhibit a similar or better viability of the yeast LALVIN QA 23 YSEO^{®} in the presence of Yeast-based Nutrient. The yeast-based nutrients had a sterol content of at least 20 mg per gram of yeast-based nutrient and was in a microgranules form.

As shown in Figure 3, the Volatile acidity of the wines resulting from the fermentation F8 and F9 in the presence of Yeast-based Nutrient exhibit lower levels of Volatile acid in wine produced. The yeast-based nutrients had a sterol content of at least 20 mg per gram of yeast-based nutrient and was in a microgranules form. The fermentation F7 (direct addition) exhibit a similar level of Volatile acid in wine produced than controls, suggesting that the direct addition of the Yeast-based Nutrient is not increasing the levels of Volatile acid in wines.

The yeast-based nutrient naturally rich or enriched in sterols (having a sterol content of at least 20 mg per gram of yeast-based nutrient), when in microgranules form, allows for a significant shorter fermentation time, therefore preventing or reducing slow and/or sluggish alcoholic fermentation and maintaining or increasing the same fermenting capacity of Active Dry Yeast (ADY) or Instant Dry Yeast (IDY) during alcoholic fermentation than similar products in powder form for example. The fermentation results shows that the microgranule form of the yeast-based nutrient having a sterol content of at least 20 mg per gram of yeast-based nutrient is very versatile and allows for utilization either in direct inoculation or in standard rehydration. Moreover, it was shown in the present description that when used in rehydration or in direct inoculation, the yeast-based nutrient having a sterol content of at least 20 mg per gram of yeast-based nutrient in a microgranule form does not require access to hot water and can be used at the temperature of the must which is generally in the range of about 10 to about 25 °Celsius.

### EXAMPLE 3: Transfer of sterols from a yeast-based nutrient in accordance with the present description to live yeast (ADYs and IDYs).

Two yeast strains Lalvin QA23TM and ECA5TM were rehydrated using the classical method known in the art (in water at a temperature of 37°C for a period of 20 minutes) and the sterol content of their respective membranes was measured after the rehydration. In parallel, a rehydration of yeast strains Lalvin QA23TM and ECA5TM was performed with the yeast-based nutrient in accordance with the present description. The yeast-based nutrient had a sterol content of at least 20 mg per gram of yeast-based nutrient and was in a microgranules form. The rehydration of the two strains was performed in water with the yeast-based nutrient at a temperature of 15°C for a period of 15 minutes. Likewise, the sterol content of their respective membranes was measured.

As shown in Fig.4 The relative content of sterols/mg of cells clearly increased in the presence of yeast-based nutrient. The yeast-based nutrients had a sterol content of at least 20 mg per gram of yeast-based nutrient and was in a microgranules form. This shows that the sterols are quickly incorporated by the living rehydrated yeast and can then protect their membranes and the cells.

### EXAMPLE 4: Maintain or increase of the fermentation profile of a Chardonnay must by the incorporation of a yeast-based nutrient

Fermentations were carried out in 30L fermenters, in parallel and in duplicates on a Chardonay must at Weincampus Neustadt, Germany. The Chardonay must have 200 mg/l of assimilable nitrogen and containing 250 g/l of fermentable sugars, 30NTU, a pH of 3,18, and a Total acidity of 4,50 g H2SO4/L. The active dry yeast used was the commercial yeast LALVIN CY3079^{™} and was inoculated in a dosage of 25g/hl. The yeast-based nutrients were added in a dosage of 30g/hl. The yeast-based nutrients had a sterol content of at least 20 mg per gram of yeast-based nutrient and was in a microgranules form (YBN01).

### Rehydration:

### Control:

The dry yeast LALVIN CY3079^{™} was rehydrated in water at 37°C for 20 minutes.

Goferm protect Evolution^{™} (GFPE).

The dry yeast LALVIN CY3079^{™} was rehydrated in water at 37°C for 20 minutes with addition of Goferm Protect Evolution^{™} (used in a dosage permitting a concentration of 30 g/hl in the must).

### YBN01 15:

The dry yeast LALVIN CY3079^{™} was rehydrated in water at 20°C for 15 minutes with addition of the yeast-based nutrient (used in a dosage permitting a concentration of 30 g/hl in the must). The yeast-based nutrients had a sterol content of at least 20 mg per gram of yeast-based nutrient and was in a microgranules form (YBN01).

### YBN01 05

The dry yeast LALVIN CY3079^{™} was rehydrated in water at 20°C for 5 minutes with addition of the yeast-based nutrient (used in a dosage permitting a concentration of 30 g/hl in the must). The yeast-based nutrients had a sterol content of at least 20 mg per gram of yeast-based nutrient and was in a microgranules form (YBN01).

### Fermentation:

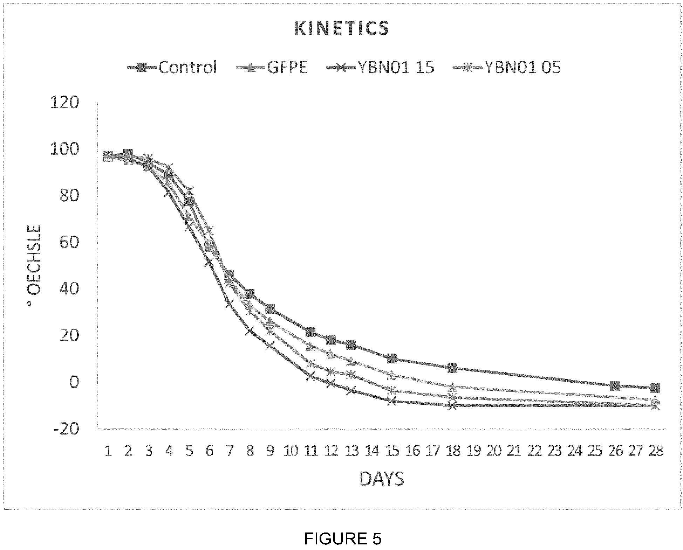

The fermentations were done at temperature between 16-20° and correspond to the following forms:
Control: Fermentation was carried out with the yeast LALVIN CY3079^{™} rehydrated in water at 37°C for 20 minutes in the absence of the yeast-based nutrient (Figure 5 Control).
GFPE: Fermentation was carried out with the yeast LALVIN CY3079^{™} rehydrated in the presence of Goferm Protect Evolution^{™} (used in a dosage permitting a concentration of 30 g/hl in the must) in the rehydration medium (Figure 5 GFPE).
YBN01 15: Fermentation carried out with the yeast LALVIN CY3079^{™} rehydrated in water at 20°C for 15 minutes in the presence of YBN01 in a dosage of 30 g/hl (Figure 5 YBN01 15). YBN01 15 was a yeast-based nutrient having a sterol content of at least 20 mg per gram of yeast-based nutrient and was in a microgranules form.
YBN01 05: Fermentation carried out with the yeast LALVIN CY3079^{™} rehydrated in water at 13°C for 5 minutes in the presence of YBN01 in a dosage of 30 g/hl (Figure 5 YBN01 5). YBN01 05 was a yeast-based nutrient having a sterol content of at least 20 mg per gram of yeast-based nutrient and was in a microgranules form.

As shown in Figure 5, the alcoholic fermentations that were carried out in the presence of Yeast-based Nutrient in accordance with the present description (YBN01 15 and YBN01 5), were completed respectively at least 14 and 10 days before the control fermentations. Moreover, Figure 5 shows that alcoholic fermentations carried out in the presence of YBN01 15 and YBN01 5, are respectively completed at least 4 and 6 days before the fermentation carried out in the presence of Goferm Protect Evolution^{™} (GFPE), a previous generation Yeast-based Nutrient in powder form disclosed in European Patent No.:1395649B2. The yeast-based nutrients naturally rich or enriched in sterols of the present description, when in microgranules form, allows for a significant shorter fermentation time than similar products in powder form for example. YBN01 15 and YBN01 05 were yeast-based nutrients having a sterol content of at least 20 mg per gram of yeast-based nutrient and was in a microgranules form.

### EXAMPLE 5: Maintain or increase of the fermentation profile of a Chardonnay must by the incorporation of a yeast-based nutrient

Fermentations were carried out in 100L fermenters, in parallel and in duplicates on a white Chardonnay must at a winery located in La Rioja, Spain. The Chardonnay must have 334 mg/l of assimilable nitrogen and containing 225 g/l of fermentable sugars, 195NTU, a pH of 3,6, and a Total acidity of 7,3 g H2SO4/L. The active dry yeast used was the commercial yeast LALVIN CY3079^{™} and was inoculated in a dosage of 25g/hl. The yeast-based nutrient was added in a dosage of 30g/hl. The yeast-based nutrient had a sterol content of at least 20 mg per gram of yeast-based nutrient and was in a microgranules form.

### Rehydration:

### Control:

The dry yeast LALVIN CY3079^{™} was rehydrated in water at 37°C for 20 minutes.

Goferm protect Evolution^{™}.

The dry yeast LALVIN CY3079^{™} was rehydrated in water at 37°C for 20 minutes with addition of Goferm Protect Evolution^{™} (used in a dosage permitting a concentration of 30 g/hl in the must).

### YBN01:

The dry yeast LALVIN CY3079^{™} was rehydrated in water at 20°C for 15 minutes with addition of the yeast-based nutrient (used in a dosage permitting a concentration of 30 g/hl in the must). The yeast-based nutrient had a sterol content of at least 20 mg per gram of yeast-based nutrient and was in a microgranules form.

### Fermentation:

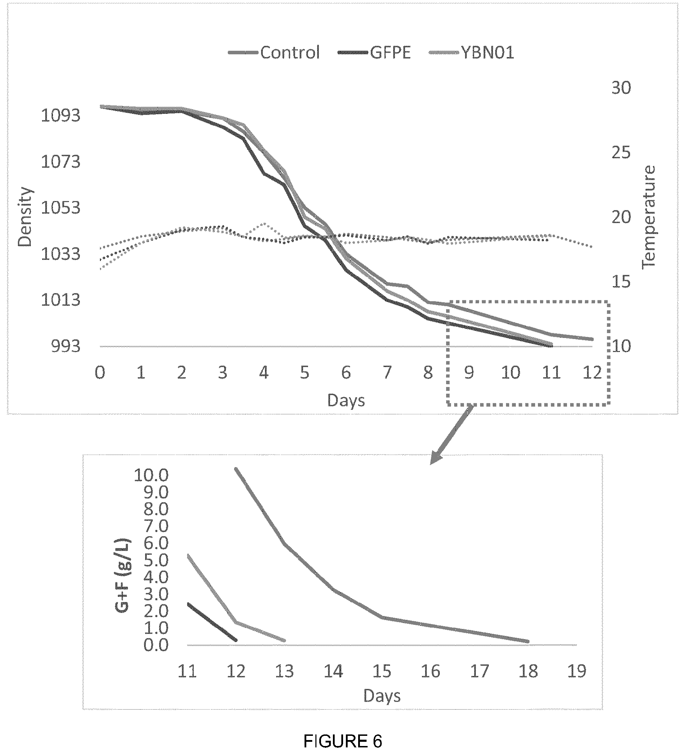

The fermentations were done at temperature between 15-18° and correspond to the following forms:
Control: Fermentation was carried out at 18°C with the yeast LALVIN CY3079^{™} rehydrated in water at 37°C for 20 minutes in the absence of the yeast-based Nutrient (Figure 6 Control).
GFPE: Fermentation was carried out at 15°C with the yeast LALVIN CY3079^{™} rehydrated in the presence of Goferm Protect Evolution^{™} (used in a dosage permitting a concentration of 30 g/hl in the must) in the rehydration medium (Figure 6 GFPE).
YBN01: Fermentation carried out at 15 °C with the yeast LALVIN CY3079^{™} rehydrated in water at 20°C for 15 minutes in the presence of YBN01 in a dosage of 30 g/hl (Figure 6 YBN01). YBN01 was a yeast-based nutrient having a sterol content of at least 20 mg per gram of yeast-based nutrient and was in a microgranules form.

As shown in Figure 6, the alcoholic fermentation that was carried out in the presence of the yeast-based Nutrient (YBN01) was completed 5 days before the control fermentation. YBN01 was a yeast-based nutrient having a sterol content of at least 20 mg per gram of yeast-based nutrient and was in a microgranules form.

Also, according to Figure 6, the alcoholic fermentation carried out in the presence of the yeast based-nutrient YBN01 was slightly slower in the final part of the alcoholic fermentation in comparison to the fermentation carried out in the presence of Goferm Protect Evolution^{™} (GFPE), a previous generation Yeast-based Nutrient in powder form disclosed in European Patent No.:1395649B2., and took an extra day, to consume the residual sugars However, the yeast-based nutrient (YBN01) has the great advantage of permitting a rehydration without having to heat the rehydration media to 37 degrees Celsius. In other words, a rehydration made with the yeast-based nutrient (YBN01) can be made at room temperature or at temperature between about 15 and about 25 degrees Celsius. The resulting rehydration medium can be used to inoculate the fermentation vessel at the temperature of the must which is generally in the range of about 10 to about 25 °Celsius, without the need to dilute progressively the rehydration medium using grape must from the tank vessel to be fermented, avoiding the possibility of a temperature shock if there is a large difference with respect to the cold must to be fermented. The yeast-based nutrient in microgranules form (YBN01) also allows for a greater solubility in the rehydration medium. YBN01 was a yeast-based nutrient having a sterol content of at least 20 mg per gram of yeast-based nutrient and was in a microgranules form

## Claims

1. A yeast-based nutrient having a sterol content of at least 20 mg per gram of yeast-based nutrient, wherein the yeast-based nutrient is in a microgranules form and is capable of maintaining or increasing fermenting capacity of an Active Dry Yeast (ADY) or an Instant Dry Yeast (IDY) during alcoholic fermentation, wherein the microgranules have a median particle size, on a particle volume basis, of from about 200 µm to about 400 µm, and wherein the yeast-based nutrient is an inactivated yeast, a yeast autolysate, a yeast cell hull, yeast extract or mixtures thereof.

2. The yeast-based nutrient of claim 1, wherein the sterol content is:
(a) at least 21 mg, 22 mg, 23 mg, 24 mg, 25 mg, 26 mg, 27 mg, 28 mg, 29 mg, 30 mg, 31 mg, 32 mg, 33 mg, 34 mg or 35 mg per gram of yeast-based nutrient; or
(b) within the range of about 20 mg to 35 mg per gram of nutrient, preferably within the range of about 25 mg to 35 mg per gram of nutrient.

3. The yeast-based nutrient of claim 1 or 2, wherein at least 50% of the microgranules have a size within the range of about 200 to about 400 µm, preferably within the range of about 200 to about 300 µm.

4. The yeast-based nutrient of any one of claims 1 to 3, wherein the yeast is (i) from a *Saccharomyces;* (ii) from *Torulaspora, Metschnikowia, Pichia, Lachancea, Starmerella, Kluyveromyces* or *Candida*; or (iii) a mixture of at least two of *Saccharomyces, Torulaspora, Metschnikowia, Pichia, Lachancea, Starmerella, Kluyveromyces* or *Candida.*

5. The yeast-based nutrient of any one of claims 1 to 4, wherein:
(a) the yeast is naturally rich in sterols or is enriched in sterols; or
(b) a sterol composition in soluble form was added to the yeast-based nutrient.

6. A method of rehydrating Active Dry Yeast (ADY) or Instant Dry Yeast (IDY), wherein the method comprises adding a yeast-based nutrient having a sterol content of at least 20 mg per g of the yeast-based nutrient and in a microgranules form to the ADY or IDY in a rehydration media at less than 37°C, wherein the microgranules have a median particle size, on a particle volume basis, of from about 200 µm to about 400 µm, and wherein the yeast-based nutrient is an inactivated yeast, a yeast autolysate, a yeast cell hull, yeast extract or mixtures thereof.

7. The method of claim 6, wherein the rehydration is performed for 20 minutes or less.

8. The method of claim 6 or 7, wherein the method further comprises performing an alcoholic fermentation of a fermentation medium, optionally wherein the fermentation medium is a wine must.

9. A method of maintaining or increasing a fermenting capacity of Active Dry Yeast (ADY) or Instant Dry Yeast (IDY) during alcoholic fermentation, the method comprises adding a yeast-based nutrient having a sterol content of at least 20 mg per gram of the yeast-based nutrient in a rehydration media with the Active Dry Yeast (ADY) or Instant Dry Yeast (IDY) to be rehydrated, or in a vessel containing the Active Dry Yeast (ADY) or Instant Dry Yeast (IDY) and a must to be fermented, wherein the yeast-based nutrient is in a microgranules form and is capable of increasing or maintaining a fermenting capacity of Active Dry Yeast (ADY) or Instant Dry Yeast (IDY) during alcoholic fermentation, wherein the microgranules have a median particle size, on a particle volume basis, of from about 200 µm to about 400 µm, and wherein the yeast-based nutrient is an inactivated yeast, a yeast autolysate, a yeast cell hull, yeast extract or mixtures thereof.

10. The method of any one of claims 6 to 9, wherein the yeast-based nutrient is added to the rehydration media, or the must to be fermented, at 10°C to less than 37°C, such as 10°C - 30°C, 10°C - 25°C or 15°C - 25°C.

11. The method of any one of claims 6 to 10, wherein the ADY or IDY is in powder, vermicelli or pellet form.

12. The method of any one of claims 6 to 11, wherein the sterol content is:
(a) at least 21 mg, 22 mg, 23 mg, 24 mg, 25 mg, 26 mg, 27 mg, 28 mg, 29 mg, 30 mg, 31 mg, 32 mg, 33 mg, 34 mg or 35 mg per gram of yeast-based nutrient; or
(b) within the range of about 20 mg to 35 mg per gram of nutrient, preferably within the range of about 25 mg to 35 mg per gram of nutrient.

13. The method of any one of claims 6 to 12, wherein at least 50% of the microgranules have a size within the range of about 200 to about 400 µm, preferably within the range of about 200 to about 300 µm.

14. The method of any one of claims 6 to 13, wherein the yeast is (i) from a *Saccharomyces*; (ii) *Torulaspora, Metschnikowia, Pichia, Lachancea, Starmerella, Kluyveromyces* or *Candida*; or (iii) a mixture of at least two of *Saccharomyces, Torulaspora, Metschnikowia, Pichia, Lachancea, Starmerella, Kluyveromyces* or *Candida.*

15. The method of any one of claims 6 to 14, wherein:
(a) the yeast is naturally rich in sterols or is enriched in sterols; or
(b) a sterol composition in soluble form was added to the yeast-based nutrient.

## Patentansprüche

1. Nährstoff auf Hefebasis mit einem Sterolgehalt von mindestens 20 mg pro Gramm des Nährstoffs auf Hefebasis, wobei der Nährstoff auf Hefebasis in Form von Mikrogranulaten vorliegt und in der Lage ist, die Gärungsfähigkeit einer aktiven Trockenhefe (Active Dry Yeast, ADY) oder einer Instant-Trockenhefe (Instant Dry Yeast, IDY) während der alkoholischen Gärung aufrechtzuerhalten oder zu steigern, wobei die Mikrogranulate eine mittlere Partikelgröße, bezogen auf das Partikelvolumen, von etwa 200 µm bis etwa 400 µm aufweisen, und wobei der Nährstoff auf Hefebasis eine inaktivierte Hefe, ein Hefeautolysat, eine Hefezellhülle, Hefeextrakt oder Gemische davon ist.

2. Nährstoff auf Hefebasis nach Anspruch 1, wobei der Sterolgehalt:
(a) mindestens 21 mg, 22 mg, 23 mg, 24 mg, 25 mg, 26 mg, 27 mg, 28 mg, 29 mg, 30 mg, 31 mg, 32 mg, 33 mg, 34 mg oder 35 mg pro Gramm des Nährstoffs auf Hefebasis beträgt; oder
(b) im Bereich von etwa 20 mg bis 35 mg pro Gramm Nährstoff liegt, vorzugsweise im Bereich von etwa 25 mg bis 35 mg pro Gramm Nährstoff.

3. Nährstoff auf Hefebasis nach Anspruch 1 oder 2, wobei mindestens 50 % der Mikrogranulate eine Größe im Bereich von etwa 200 bis etwa 400 µm, vorzugsweise im Bereich von etwa 200 bis etwa 300 µm, aufweisen.

4. Nährstoff auf Hefebasis nach einem der Ansprüche 1 bis 3, wobei die Hefe (i) aus einer *Saccharomyces-Art* stammt; (ii) aus *Torulaspora, Metschnikowia, Pichia, Lachancea, Starmerella, Kluyveromyces* oder *Candida* stammt; oder (iii) ein Gemisch aus mindestens zwei der Gattungen *Saccharomyces, Torulaspora, Metschnikowia, Pichia, Lachancea, Starmerella, Kluyveromyces* oder *Candida* ist.

5. Nährstoff auf Hefebasis nach einem der Ansprüche 1 bis 4, wobei:
(a) die Hefe von Natur aus reich an Sterolen ist oder mit Sterolen angereichert ist; oder
(b) dem Nährstoff auf Hefebasis eine Sterolzusammensetzung in löslicher Form zugesetzt wurde.

6. Verfahren zum Rehydrieren von aktiver Trockenhefe (ADY) oder Instant-Trockenhefe (IDY), wobei das Verfahren das Hinzufügen eines Nährstoffs auf Hefebasis mit einem Sterolgehalt von mindestens 20 mg pro g des Nährstoffs auf Hefebasis und in Form von Mikrogranulaten zu der ADY oder IDY in einem Rehydrierungsmedium bei weniger als 37 °C umfasst, wobei die Mikrogranulate eine mittlere Partikelgröße, bezogen auf das Partikelvolumen, von etwa 200 µm bis etwa 400 µm aufweisen, und wobei der Nährstoff auf Hefebasis eine inaktivierte Hefe, ein Hefeautolysat, eine Hefezellhülle, Hefeextrakt oder Gemische davon ist.

7. Verfahren nach Anspruch 6, wobei die Rehydrierung für 20 Minuten oder weniger durchgeführt wird.

8. Verfahren nach Anspruch 6 oder 7, wobei das Verfahren ferner die Durchführung einer alkoholischen Gärung eines Gärmediums umfasst, optional wobei das Gärmedium ein Weinmost ist.

9. Verfahren zur Aufrechterhaltung oder Steigerung der Gärungsfähigkeit von aktiver Trockenhefe (ADY) oder Instant-Trockenhefe (IDY) während der alkoholischen Gärung, wobei das Verfahren das Hinzufügen eines Nährstoffs auf Hefebasis mit einem Sterolgehalt von mindestens 20 mg pro Gramm des Nährstoffs auf Hefebasis in ein Rehydrierungsmedium mit der zu rehydrierenden aktiven Trockenhefe (ADY) oder Instant-Trockenhefe (IDY), die rehydriert werden soll, oder in einem Gefäß, das die aktive Trockenhefe (ADY) oder Instant-Trockenhefe (IDY) und einen zu vergärenden Most enthält, umfasst, wobei der Nährstoff auf Hefebasis in Form von Mikrogranulaten vorliegt und in der Lage ist, die Gärungsfähigkeit einer aktiven Trockenhefe (ADY) oder einer Instant-Trockenhefe (IDY) während der alkoholischen Gärung zu steigern oder aufrechtzuerhalten, wobei die Mikrogranulate eine mittlere Partikelgröße, bezogen auf das Partikelvolumen, von etwa 200 µm bis etwa 400 µm aufweisen, und wobei der Nährstoff auf Hefebasis eine inaktivierte Hefe, ein Hefeautolysat, eine Hefezellhülle, Hefeextrakt oder Gemische davon ist.

10. Verfahren nach einem der Ansprüche 6 bis 9, wobei der Nährstoff auf Hefebasis dem Rehydrationsmedium oder dem zu vergärenden Most bei 10 °C bis unter 37 °C, beispielsweise bei 10 °C bis 30 °C, 10 °C bis 25 °C oder 15 °C bis 25 °C, zugesetzt wird.

11. Verfahren nach einem der Ansprüche 6 bis 10, wobei die ADY oder IDY in Pulver-, Faden- oder Pelletform vorliegt.

12. Verfahren nach einem der Ansprüche 6 bis 11, wobei der Sterolgehalt:
(a) mindestens 21 mg, 22 mg, 23 mg, 24 mg, 25 mg, 26 mg, 27 mg, 28 mg, 29 mg, 30 mg, 31 mg, 32 mg, 33 mg, 34 mg oder 35 mg pro Gramm des Nährstoffs auf Hefebasis beträgt; oder
(b) im Bereich von etwa 20 mg bis 35 mg pro Gramm Nährstoff liegt, vorzugsweise im Bereich von etwa 25 mg bis 35 mg pro Gramm Nährstoff.

13. Verfahren nach einem der Ansprüche 6 bis 12, wobei mindestens 50 % der Mikrogranulate eine Größe im Bereich von etwa 200 bis etwa 400 µm, vorzugsweise im Bereich von etwa 200 bis etwa 300 µm, aufweisen.

14. Verfahren nach einem der Ansprüche 6 bis 13, wobei die Hefe (i) aus einer *Saccharomyces*-Art stammt; (ii) aus *Torulaspora, Metschnikowia, Pichia, Lachancea, Starmerella, Kluyveromyces* oder *Candida* stammt; oder (iii) ein Gemisch aus mindestens zwei der Gattungen *Saccharomyces, Torulaspora, Metschnikowia, Pichia, Lachancea, Starmerella, Kluyveromyces* oder *Candida* ist.

15. Verfahren nach einem der Ansprüche 6 bis 14, wobei:
(a) die Hefe von Natur aus reich an Sterolen ist oder mit Sterolen angereichert ist; oder
(b) dem Nährstoff auf Hefebasis eine Sterolzusammensetzung in löslicher Form zugesetzt wurde.

## Revendications

1. Nutriment à base de levure, ayant une teneur en stérol d'au moins 20 mg par gramme de nutriment à base de levure, dans lequel le nutriment à base de levure est sous forme de microgranules et est capable de maintenir ou d'augmenter une capacité de fermentation d'une levure sèche active (Active Dry Yeast, ADY) ou d'une levure sèche à action rapide (Instant Dry Yeast, IDY) durant une fermentation alcoolique, dans lequel les microgranules ont une taille particulaire médiane, sur une base volumique particulaire, d'environ 200 µm à environ 400 µm, et dans lequel le nutriment à base de levure est une levure inactivée, un autolysat de levure, une enveloppe cellulaire de levure, un extrait de levure, ou des mélanges de ceux-ci.

2. Nutriment à base de levure de la revendication 1, dans lequel la teneur en stérol est :
(a) d'au moins 21 mg, 22 mg, 23 mg, 24 mg, 25 mg, 26 mg, 27 mg, 28 mg, 29 mg, 30 mg, 31 mg, 32 mg, 33 mg, 34 mg ou 35 mg par gramme de nutriment à base de levure ; ou
(b) dans les limites de la plage d'environ 20 mg à 35 mg par gramme de nutriment, de préférence dans les limites de la plage d'environ 25 mg à 35 mg par gramme de nutriment.

3. Nutriment à base de levure de la revendication 1 ou 2, dans lequel au moins 50 % des microgranules ont une taille dans les limites de la plage d'environ 200 à environ 400 µm, de préférence dans les limites de la plage d'environ 200 à environ 300 µm.

4. Nutriment à base de levure de l'une quelconque des revendications 1 à 3, dans lequel la levure (i) provient d'un *Saccharomyces* ; (ii) provient de : *Torulaspora, Metschnikowia, Pichia, Lachancea, Starmerella, Kluyveromyces,* ou *Candida* ; ou (iii) est un mélange d'au moins deux de : *Saccharomyces, Torulaspora, Metschnikowia, Pichia, Lachancea, Starmerella, Kluyveromyces,* ou *Candida.*

5. Nutriment à base de levure de l'une quelconque des revendications 1 à 4, dans lequel :
(a) la levure est naturellement riche en stérols ou est enrichie en stérols ; ou
(b) une composition de stérol sous forme soluble a été ajoutée au nutriment à base de levure.

6. Procédé de réhydratation de levure sèche active (Active Dry Yeast, ADY) ou de levure sèche à action rapide (Instant Dry Yeast, IDY), dans lequel le procédé comprend l'ajout d'un nutriment à base de levure, ayant une teneur en stérol d'au moins 20 mg par g du nutriment à base de levure et sous une forme de microgranules, à la ADY ou la IDY dans un milieu de réhydratation à moins de 37 °C, dans lequel les microgranules ont une taille particulaire médiane, sur une base volumique particulaire, d'environ 200 µm à environ 400 µm, et dans lequel le nutriment à base de levure est une levure inactivée, un autolysat de levure, une enveloppe cellulaire de levure, un extrait de levure, ou des mélanges de ceux-ci.

7. Procédé de la revendication 6, dans lequel la réhydratation est réalisée pendant 20 minutes ou moins.

8. Procédé de la revendication 6 ou 7, dans lequel le procédé comprend en outre la réalisation d'une fermentation alcoolique d'un milieu de fermentation, facultativement dans lequel le milieu de fermentation est un moût de vin.

9. Procédé de maintien ou d'augmentation d'une capacité de fermentation de levure sèche active (Active Dry Yeast, ADY) ou de levure sèche à action rapide (Instant Dry Yeast, IDY) durant une fermentation alcoolique, le procédé comprend l'ajout d'un nutriment à base de levure, ayant une teneur en stérol d'au moins 20 mg par gramme du nutriment à base de levure, dans un milieu de réhydratation avec la levure sèche active (ADY) ou levure sèche à action rapide (IDY) destinée à être réhydratée, ou dans un récipient contenant la levure sèche active (ADY) ou la levure sèche à action rapide (IDY) et un moût destiné à être fermenté, dans lequel le nutriment à base de levure est sous forme de microgranules et est capable d'augmenter ou de maintenir une capacité de fermentation de levure sèche active (ADY) ou de levure sèche à action rapide (IDY) durant une fermentation alcoolique, dans lequel les microgranules ont une taille particulaire médiane, sur une base volumique particulaire, d'environ 200 µm à environ 400 µm, et dans lequel le nutriment à base de levure est une levure inactivée, un autolysat de levure, une enveloppe cellulaire de levure, un extrait de levure, ou des mélanges de ceux-ci.

10. Procédé de l'une quelconque des revendications 6 à 9, dans lequel le nutriment à base de levure est ajouté au milieu de réhydratation, ou au moût destiné à être fermenté, à 10 °C à moins de 37 °C, par exemple de 10 °C à 30 °C, de 10 °C à 25 °C, ou de 15 °C à 25 °C.

11. Procédé de l'une quelconque des revendications 6 à 10, dans lequel la ADY ou la IDY est sous forme de poudre, de vermicelle, ou de pastille.

12. Procédé de l'une quelconque des revendications 6 à 11, dans lequel la teneur en stérol est :
(a) d'au moins 21 mg, 22 mg, 23 mg, 24 mg, 25 mg, 26 mg, 27 mg, 28 mg, 29 mg, 30 mg, 31 mg, 32 mg, 33 mg, 34 mg ou 35 mg par gramme de nutriment à base de levure ; ou
(b) dans les limites de la plage d'environ 20 mg à 35 mg par gramme de nutriment, de préférence dans les limites de la plage d'environ 25 mg à 35 mg par gramme de nutriment.

13. Procédé de l'une quelconque des revendications 6 à 12, dans lequel au moins 50 % des microgranules ont une taille dans les limites de la plage d'environ 200 à environ 400 µm, de préférence dans les limites de la plage d'environ 200 à environ 300 µm.

14. Procédé de l'une quelconque des revendications 6 à 13, dans lequel la levure est (i) provient d'un *Saccharomyces* ; (ii) *Torulaspora, Metschnikowia, Pichia, Lachancea, Starmerella, Kluyveromyces* ou *Candida* ; ou (iii) est un mélange d'au moins deux de : *Saccharomyces, Torulaspora, Metschnikowia, Pichia, Lachancea, Starmerella, Kluyveromyces,* ou *Candida.*

15. Procédé de l'une quelconque des revendications 6 à 14, dans lequel :
(a) la levure est naturellement riche en stérols ou est enrichie en stérols ; ou
(b) une composition de stérol sous forme soluble a été ajoutée au nutriment à base de levure.
